# EUROPEAN PATENT APPLICATION

(11) **EP 4 390 968 A1**
(43) Date of publication of application: **26.06.2024**
(21) Application number: 22215142.5
(22) Date of filing: 20.12.2022
(51) Int. Cl.: G16H 50/50, G16H 20/30

(54) **SYSTEM AND METHOD FOR BUILDING DYNAMIC BIOMECHANICAL PROFILES OF PERSONS**

(71) Applicant: University of Pisa, 56121 Pisa (IT); University of Strathclyde, Glasgow G1 1XJ (GB); University of Malta, Msida MSD 2080 (MT)
(72) Inventor: RAZIONALE, Armando V., 56121 Pisa (IT); WODEHOUSE, Andrew, Glasgow, G1 1XJ (GB); FARRUGIA, Philip, Msida, MSD2080 (MT); PAOLI, Alessandro, 56121 Pisa (IT); NERI, Paolo, 56121 Pisa (IT); TAMBURRINO, Francesco, 56121 Pisa (IT); ARUANNO, Beatrice, 56121 Pisa (IT); BALZAN, Emanuel, Msida, MSD2080 (MT); ABELA, Edward, Msida, MSD2080 (MT); URQUHART, Lewis, Glasgow, G1 1XJ (GB)
(74) Representative: De Ros, Alberto

(57) **Abstract**

An embodiment of the innovative system (100) allows to build a dynamic biomechanical profile (170) of a patient (10) relating to an arm of the patient (10); the system (100) comprises: first means (110) arranged to detect a shape of the arm and to generate shape data, second means (120) arranged to measure movements possible for the arm and to generate motion data, third means (130) arranged to measure forces produced by the arm and to generate force data, and a computerized processing unit (140) arranged to receive said shape data, said motion data and said force data and to merge said data into a dynamic biomechanical profile (170) of the patient (10). In general, the innovative system and method may be applied to a whole body or a portion of body of a person.

## Description

### TECHNICAL FIELD

The subject matter disclosed herein relates to a system and a method for building a dynamic biomechanical profile of a person, for example a patient, relating in particular to his/her arm, i.e. a portion of his body.

### BACKGROUND ART

It is common practice of doctors to assess their patients in order to determine health status including impairments and disabilities. This is often supplemented by examinations, e.g. blood, radiographic, and ecographic examinations.

The ultimate goal is to choose appropriate treatments for patients (if necessary), to apply treatments to patients and to check effectiveness of treatments till healing or recovery or at least (satisfactory) improvement.

According to some embodiments, the subject matter disclosed herein focusses on upper limb impairments and disabilities, while according to other embodiments, the subject matter disclosed herein focusses on lower limb impairments and disabilities. The treatments considered are essentially therapeutical and/or rehabilitation and/or habilitation exercises.

In this specific field, it is common practice that a doctor assesses manually, often by means of specific tools, possibilities of movement of the upper limb and/or its components (shoulder, upper arm, elbow, lower arm, wrist, hand, fingers) and the forces that the upper limb and/or its components may develop and exert.

It would desirable to improve such common practice.

### SUMMARY

According to a first main aspect, the subject matter disclosed herein relates to a system for building a dynamic biomechanical profile of a patient relating to an arm of the patient as set out in independent claim 1.

According to a second main aspect, the subject matter disclosed herein relates to a computer-implemented method for building a dynamic biomechanical profile of a person relating to a body or portion of body of the person as set out in independent claim 13.

A single (i.e. integrated) dynamic biomechanical profile may be very useful; advantageously, the dynamic biomechanical profile is obtained easily and/or in a reliable way and/or in a short time and/or in a single assessment session. For example, it may be useful for assessing a patient repeatedly/periodically in order to check treatment(s) effectiveness, and/or for designing patient treatments (in particular therapeutical and/or rehabilitation and/or habilitation exercises), and/or for designing a customized therapeutic and/or rehabilitation and/or habilitation medical device.

It is to be noted that the dynamic biomechanical profile of a body or portion of body of a person may also be embodied in a 3D dynamic biomechanical model of the body or portion of body which is advantageously a digital twin of the body or portion of body.

A possible application of the subject matter disclosed herein, in particular of the dynamic biomechanical profile, is in the field of video games (in a broad sense, i.e. including games for entertainment, educational, therapeutical, rehabilitation, habilitation and other purposes).

In fact, others important aspects disclosed herein are a software component for a video game in the form of an avatar and a video game as set out in claims 14 and 15.

### BRIEF DESCRIPTION OF THE DRAWINGS

A more complete appreciation of the disclosed embodiments of the invention and many of the attendant advantages thereof will be readily obtained as the same become better understood by reference to the following detailed description when considered in connection with the accompanying drawing, wherein:

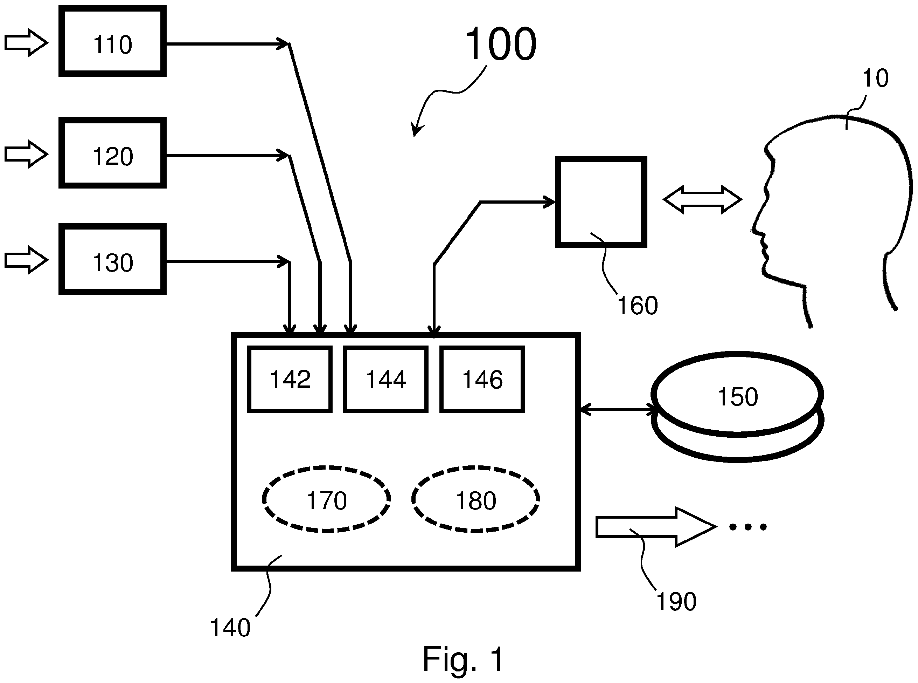

Fig. 1 shows a schematic block diagram of an embodiment of an innovative system for building a dynamic biomechanical profile.

### DETAILED DESCRIPTION OF EMBODIMENTS

According to the prior art, shape data, extent-of-motion data, and force data of a person are collected separately, maintained/stored separately, and used separately. A single dynamic (i.e. incorporating not only shape information but also cinematic information as well as dynamic information) biomechanical profile of a person integrating shape data, motion data (including speed data and not only extent-of-motion data), and force data, may be very useful in various applications, particularly but not exclusively medical applications (in this case, the person may be called "patient").

In order to simplify the profile, it is advantageous to consider instead of the whole body of the person a portion of body of the person; the following description will consider the "upper limb", commonly called "arm" but this should not be construed a limitation of the present invention. The following embodiments apply in particular to any combination of components of the upper limb (shoulder, upper arm, elbow, lower arm, wrist, hand, fingers).

Reference now will be made in detail to embodiments of the disclosure, an example of which is illustrated in the drawing. The example and the drawing figure are provided by way of explanation of the disclosure and should not be construed as a limitation of the disclosure. In fact, it will be apparent to those skilled in the art that various modifications and variations can be made in the present disclosure without departing from the scope or spirit of the disclosure. It is to be noted that several features described separately are independent between each other but may be combined to form particularly advantageous embodiments.

Fig. 1 shows an embodiment of a system 100 for building a dynamic biomechanical profile of a patient relating to an arm of the patient. In this figure, a person 10 is schematically shown that interacts with system 100. In this case, person 10 is both the patient whose dynamic biomechanical profile is to be built by system 100 and a user of system 100 who is interested in any function performed by system 100 (for example, displaying the dynamic biomechanical profile and/or a dynamic biomechanical model derived from the dynamic biomechanical profile). In general, the person to be observed and the user(s) are different. System 100 comprises: means 110, i.e. "first means", for generating shape data, means 120, i.e. "second means", for generating motion data, means 130, i.e. "third means", for generating force data, and a computerized processing unit 140 arranged to receive at least shape data, motion data and force data, to process them (if necessary) and to merge them into a single (i.e. integrated) dynamic biomechanical profile of the patient. A dynamic biomechanical profile 170 is shown in Fig. 1 surrounded by a dashed line as a result of a processing internal to unit 140, i.e. not a HW (= Hardware) component.

Computerized processing unit 140 may have a conventional HW structure, for example it may be a PC with embedded operating system, with an electronic processor 142, for example a micro-processor, a memory 144 for one or more running computer programs and a memory 146 for under-processing data. The specificity of unit 140 lies in the software that allows inter alia to build dynamic biomechanical profiles and that is in memory 144 during execution and in a mass storage (not shown in Fig. 1) before and after execution.

First means 110 is arranged to detect a shape of the arm and to generate shape data. Second means 120 is arranged to measure movements (for example their linear and/or angular displacement and/or their speed and/or their acceleration) possible for the arm and to generate motion data. Third means 130 is arranged to measure forces produced by the arm and to generate force data. Such means are known per se even if, according to the prior art, commonly used separately from each other; therefore, no further details will be provided herein. It is to be noted that, according to the prior art, motion measuring means and force measuring means are tools manually used by technicians, e.g. doctors.

In Fig. 1, a storage unit 150 is shown external to processing unit 140; however, alternatively, the storage unit, for example a hard disk, may be internal to the processing unit.

The first means 110 may be arranged to transmit the shape data directly to unit 140. The shape data may be in the form of a points cloud preferably deriving from merging e.g. three separate points clouds. The shape data may be captured by asking the patient to keep the arm still (to the extent possible).

The second means 120 may be arranged to transmit the motion data directly to unit 140. The motion data may be in the form of a set of angular ranges of motion for predetermined motions of the arm such as: wrist flexion, wrist extension, wrist radial deviation, wrist ulnar deviation, wrist pronation, wrist supination, finger flexion, finger extension (i.e. extent-of-motion data) and/or a set of a list of values of motion speed limits (i.e. speed data); accordingly, in general, the term "motion data" is not to be construed imitatively, and may include in particular extent-of-motion data and/or speed data and/or acceleration data. The motion data may be organized according to a formatted JSON file. The motion data may be captured by asking the patient to move the arm appropriately (to the extent possible).

The third means 130 may be arranged to transmit the force data directly to unit 140. The force data may be in the form of a list of values for predetermined forces of the arm such as: grip force, pinch force, wrist torque. The force data may be captured by asking the patient to apply the desired force to a corresponding appropriate dynamometer (to the extent possible).

Advantageously, the innovative system (e.g. system 100) may be arranged to generate the shape data, the motion data and the force data in a single patient assessment session, for example following a predetermined assessment protocol. Unit 140 is typically arranged to store the dynamic biomechanical profiles (e.g. profile 170) after building them for future use; for example, the profiles may be stored in unit 150. Possibly, the profiles may be stored in a secure way (for example encrypted) as they represent "sensitive data". Unit 140 is typically arranged to export the dynamic biomechanical profiles (e.g. profile 170) after building them for use by other units and/or systems; in general, data output from unit 140 is represented by arrow 190 in Fig. 1. Possibly, the profiles may be exported in a secure way (for example encrypted) as they represent "sensitive data". Advantageously, unit 140 offers both storage and exportation possibilities; typically, a dynamic biomechanical profile (e.g. profile 170) is first stored just (e.g. few seconds) after being built and then (retrieved and) exported e.g. hours or days after being built.

Advantageously, unit 140 is arranged to create a dynamic biomechanical model (e.g. model 180) of the arm of the patient starting from the dynamic biomechanical profile (e.g. profile 170). This dynamic biomechanical model may be considered a so-called a "digital twin" (with a certain accuracy) of the arm of the patient. A dynamic biomechanical model 180 (being a 3D dynamic model) is shown in Fig. 1 surrounded by a dashed line as a result of a processing internal to unit 140, i.e. not a HW component. Therefore, another possible specificity of unit 140 lies in the software that allows i.a. to create dynamic biomechanical models and that is in memory 144 during execution and in a mass storage (not shown in Fig. 1) before and after execution.

Unit 140 is typically arranged to store the dynamic biomechanical models (e.g. model 180) after creating them for future use; for example, the models may be stored in unit 150. Possibly, the models may be stored in a secure way (for example encrypted) as they may be considered to represent "sensitive data". Unit 140 is typically arranged to export the dynamic biomechanical models (e.g. model 180) after creating them for use by other units and/or systems; in general, data output from unit 140 is represented by arrow 190 in Fig. 1. Possibly, the models may be exported in a secure way (for example encrypted) as they may be considered to represent "sensitive data". Advantageously, unit 140 offers both storage and exportation possibilities; a dynamic biomechanical model (e.g. model 180) is first stored just (e.g. few seconds) after being created and then (retrieved and) exported e.g. hours or days after being built.

Advantageously, the created dynamic biomechanical model is based on a model structure; for example, an arm may be considered a set of bones, muscles and tendons appropriately interconnected between each other and surrounded by a skin linked someway to the muscles. Unit 140 is arranged to determine, in particular through appropriate computations, parameters and/or attributes of the model structure from the data of the dynamic biomechanical profile.

Unit 140 may be arranged to display statically and/or dynamically a created dynamic biomechanical model for example graphically on a screen. In Fig. 1, a I/O interface 160 is schematically shown interconnected to unit 140; typically, it comprises a keyboard, a mouse or joystick and a screen. Some software, e.g. a GUI (= Graphic User Interface), may be considered part of interface 160. In Fig. 1, interface 160 is shown external to unit 140; however, alternatively, the interface may be partially or totally internal to the unit.

System 100 may be arranged to build a set of dynamic biomechanical profiles of the same arm of the same patient at time distance for example during a therapy period. Unit 140 may be arranged to compare the dynamic biomechanical profiles of this set. In this way, it is possible to evaluate the effectiveness of a therapy in a very objective way. In this case, unit 140 may be advantageously arranged to display statically and/or dynamically differences between these dynamic biomechanical profiles - reference may be made to interface 160 for this purpose. For example, if a dynamic biomechanical model is created starting from each of these dynamic biomechanical profiles, the differences between the profiles in terms of shape and/or motion and/or force may be shown very effectively for example graphically on a screen.

Unit 140 may be arranged to assist in designing patient treatments, in particular therapeutical and/or rehabilitation and/or habilitation exercises, based on a built dynamic biomechanical profile; these exercises may be even in the form of virtual-reality or augmented-reality games. In this case, unit 140 may be advantageously arranged to display statically and/or dynamically the therapeutical and/or rehabilitation and/or habilitation exercises - reference may be made to interface 160 for this purpose. For example, if a dynamic biomechanical model of the patient is created starting from the dynamic biomechanical profile of the patient, an exercise may be shown very effectively for example graphically on a screen as it should be executed just by this patient through "simulation".

Unit 140 may be arranged to assist in designing a therapeutic and/or rehabilitation and/or habilitation medical device customized for a patient based on his dynamic biomechanical profile at a specific moment, e.g. a date or week. In this case, the design assistance may advantageously include displaying statically and/or dynamically a dynamic biomechanical model possibly in combination with the medical device under design and check effectiveness of the medical device.

For this application (i.e. designing therapeutic and/or rehabilitation and/or habilitation medical devices), unit 140 may be arranged to map the built dynamic biomechanical profile into ergonomic and functional requirements for a customized therapeutic and/or rehabilitation and/or habilitation medical device. In this case, unit 140 may be further arranged to output a product design specifications file. Designing patient treatments (in particular therapeutical and/or rehabilitation and/or habilitation exercises) and/or designing therapeutic and/or rehabilitation and/or habilitation medical devices may be carried out directly by an innovative profile building system or by a system external to an innovative profile building system. In the latter case, data and/or information are exported by innovative profile building system (e.g. system 100); this is schematically shown by arrow 190 in Fig. 1.

The innovative profile building system, with all its different possibilities, as described above reflects in a corresponding innovative profile building method that is specifically conceived for being implemented through a computer.

In general, such computer-implemented method for building a dynamic biomechanical profile of a person relating to a body or portion of body of the person comprises the steps of:
detecting a shape of the body or portion of body and generating shape data, measuring movements (for example their linear and/or angular displacement and/or their speed and/or their acceleration) possible for the body or portion of body and generating motion data,
measuring forces produced by the body or portion of body and generating force data, and
merging said shape data, said motion data and said force data into a dynamic biomechanical profile of the person.

Based on the above description of the innovative profile building system, other functional features of the innovative profile building method are apparent and may be derived.

A detailed description has just been provided of a medical application of the innovative profile building system and method. However, other applications are possible. In the following, some information will be provided relating to an advantageous application in the field of video games.

Nowadays, it is common to use so-called "avatar", in particular 3D avatar, in video games; an avatar is a graphical representation of a user or the user's character or persona. Sometimes, the avatars have the appearance of famous persons, e.g. football player(s), or of the gamer(s); this typically applies only to the face while the body is completely fancy not only in terms of shape but also in terms of motion and force which is considered desirable.

Through dynamic biomechanical profiles it would be possible to create more realistic avatars, particularly but not exclusively 3D avatars, not only in terms of shape, but also (and most importantly) in terms of motion and force.

It is to be understood that similarity may refer not necessarily to the whole body but only to one or more portions of the body (e.g. the arms or the arms and the legs).

It is also to be understood that herein the term "video game" is not to be construed imitatively. In fact, some games have a purely entertainment purpose, but others have for example an educational, therapeutical, rehabilitation, and habilitation purpose.

In practice, an avatar may be considered a "software component" of a video game. Often avatars are in a way embedded in video games but are created prior to playing the video game; avatars creation may occur A) when programming the video game, and/or B) just before playing a game, and/or C) well before playing a game and stored.

An innovative avatar is based for example on a dynamic biomechanical profile of a real person (in particular a user or player of the video game); such dynamic biomechanical profile may comprise shape data and/or motion data and/or force data (preferably but not necessarily all such data) obtained through e.g. the innovative method prior to using the avatar for playing the video game.

It is to be noted that, according to some embodiments, avatar creation may be carried out by a software tool separate from the video game and, for example, associated to one or more video games.

Once one (or more) dynamic biomechanical profile has been built, then an avatar may be created based on this dynamic biomechanical profile, and finally the avatar may be integrated into a video game so an innovative video game is provided.

## Claims

1. A system (100) for building a dynamic biomechanical profile (170) of a patient (10) relating to an arm of the patient (10), wherein the system (100) comprises:
first means (110) arranged to detect a shape of the arm and to generate shape data, second means (120) arranged to measure movements possible for the arm and to generate motion data,
third means (130) arranged to measure forces produced by the arm and to generate force data, and
a computerized processing unit (140) arranged to receive said shape data, said motion data and said force data and to merge said data into a single dynamic biomechanical profile (170) of the patient (10).

2. The system (100) of any claim 1, wherein said unit (140) is arranged to create a dynamic biomechanical model (180) of the arm of the patient (10) starting from said dynamic biomechanical profile (170), said dynamic biomechanical model (180) being a digital twin of the arm of the patient.

3. The system (100) of claim 2, wherein said dynamic biomechanical model (180) is based on a model structure, and wherein said unit (140) determines parameters and/or attributes of the model structure from the data of said dynamic biomechanical profile (170).

4. The system (100) of claim 2 or 3, wherein said unit (140) is arranged to display (160) statically and/or dynamically said dynamic biomechanical model (180).

5. The system (100) of any preceding claims, wherein said system (100) is arranged to build a set of dynamic biomechanical profiles of the same arm of the same patient at time distance and wherein said unit (140) is arranged to compare the dynamic biomechanical profiles of said set.

6. The system (100) of claim 5, wherein said unit (140) is arranged to display (160) statically and/or dynamically differences between dynamic biomechanical profiles of said set, in particular through a dynamic biomechanical model (180) of the arm of the patient.

7. The system (100) of any preceding claims, wherein said unit (140) is arranged to assist in designing patient treatments, in particular therapeutical and/or rehabilitation and/or habilitation exercises, based on said dynamic biomechanical profile (170).

8. The system (100) of claim 7, wherein said unit (140) is arranged to display (160) statically and/or dynamically said therapeutical and/or rehabilitation and/or habilitation exercises in particular in the form of virtual-reality or augmented-reality games, in particular through a dynamic biomechanical model (180) of the arm of the patient.

9. The system (100) of any preceding claims, wherein said unit (140) is arranged to assist in designing a therapeutic and/or rehabilitation and/or habilitation medical device customized for said arm of said patient based on said dynamic biomechanical profile (170).

10. The system (100) of claim 9, wherein the design assistance includes displaying (160) statically and/or dynamically a dynamic biomechanical model (180) of said arm of said patient.

11. The system (100) of any preceding claims, wherein said unit (140) is arranged to map said dynamic biomechanical profile (170) into ergonomic and functional requirements for a customized therapeutic and/or rehabilitation and/or habilitation medical device.

12. The system (100) of claim 11, wherein said unit (140) is arranged to output (190) a product design specifications file.

13. A computer-implemented method for building a dynamic biomechanical profile of a person relating to a body or portion of body of the person, wherein the method comprises the steps of:
detecting a shape of the body or portion of body and generating shape data,
measuring movements possible for the body or portion of body and generating motion data,
measuring forces produced by the body or portion of body and generating force data, and
merging said shape data, said motion data and said force data into a dynamic biomechanical profile of the person.

14. A software component for a video game in the form of an avatar, wherein the avatar is based on a dynamic biomechanical profile of a real person, wherein the dynamic biomechanical profile comprises shape data, motion data and force data obtained through the method of claim 13 prior to using the avatar for playing the video game.

15. A video game comprising the software component of claim 14.
